# EUROPEAN PATENT APPLICATION

(11) **EP 3 527 982 A1**
(43) Date of publication of application: **21.08.2019**
(21) Application number: 17862541.4
(22) Date of filing: 26.09.2017
(51) Int. Cl.: G01N 33/50, A61K 35/28

(54) **METHOD FOR SELECTING HIGH-EFFICACY STEM CELL BY USING DOWNREGULATION IN EXPRESSION OR ACTIVITY OF SOCS**

(30) Priority: 17.10.2016 KR 20160134082
(71) Applicant: Samsung Life Public Welfare Foundation, Seoul 04348 (KR)
(72) Inventor: YOO, Keon Hee, Seoul 06191 (KR); LEE, Myoung Woo, Seoul 05614 (KR); KIM, Dae Seong, Goyang-si Gyeonggi-do 10422 (KR)
(74) Representative: Gulde & Partner
(86) International application number: PCT/KR2017/010591
(87) International publication number: WO 2018/074757

(57) **Abstract**

The present invention relates to a method for selecting high-efficacy mesenchymal stem cells for use in treating an immune disease, the method comprising a step of measuring a level of a particular immunosuppressive biomarker in mesenchymal stem cells in which the expression or activity of suppressor of cytokine signaling (SOCS) is downregulated, high-efficacy mesenchymal stem cells selected by the method, and a method for treating an immune disease by using the high-efficacy mesenchymal stem cells. Providing a method useful for acquiring functionally excellent mesenchymal stem cells having an ability to control immune reactions for clinical treatment of various immune diseases including graft-versus-host diseases and autoimmune diseases, the present invention can find useful applications in the therapy of immune diseases.

## Description

### [Technical Field]

The present invention relates to a method for selecting high-efficiency mesenchymal stem cells to treat an immune disease, which includes measuring a level of a particular immunosuppressive biomarker in mesenchymal stem cells in which the expression or activity of a suppressor of cytokine signaling (SOCS) is downregulated, high-efficiency mesenchymal stem cells selected by the method, and a method for treating an immune disease using the high-efficiency mesenchymal stem cells.

### [Background Art]

Organ, tissue or cell transplantation may be used to save the lives of patients suffering from various types of diseases. Allotransplantation for human organs such as kidney, liver, heart, lung, pancreas and the like, tissue such as skin and cells such bone marrow cells is a solution to treatment of an intractable disease such as terminal organ failure that is generally performed in clinics. In addition, xenotransplantation with a non-human mammal as a donor is a solution to donor shortage for allotransplantation, and is actively being studied. Particularly, in recent years, transplantation of stem cells capable of permanently regenerating themselves and differentiating into various types of cells under suitable conditions has attracted attention as substitute therapy for treating various intractable diseases.

Generally, the normal immune system of a recipient having normal function recognizes a transplanted organ, tissue or cells as "non-self," and induces an immune rejection response to the graft. The immune rejection is generally mediated by alloreactive or xenoreactive T cells present in the immune system of a recipient recognizing alloantigens or xenoantigens of donor tissue. Therefore, in order to for an allograft or xenograft to survive for a long time, the immune system of the recipient should be prevented from recognizing a foreign antigen or an immune response should be inhibited.

Graft-versus-host disease is induced by a donor's T cells activated by antigen-presenting cells of a host, and the cells migrate to target tissues (the skin, intestines, and liver), thereby inducing the dysfunction of a target organ. The first-line standard therapy of graft-versus-host disease is treatment with a high concentration of steroids. However, 50% of the patients are not responsive to the first-line therapy, and once steroid resistance is induced, it is known that mortality increases, and there is no further therapeutic alternative because there is no second-line therapy for steroid-resistant graft-versus-host disease.

In addition, to prevent a recipient's immune response to a graft, an immunosuppressive drug is usually administered to the recipient. Representative immunosuppressive drugs include calcineurin inhibitors such as cyclosporin and tacrolimus (FK-506), and antiproliferative drugs such as azathioprine, rapamycin, mycophenolate mofetil and cyclophosphamide, which are currently frequently used in allotransplantation for the kidney, liver, pancreas or heart.

These types of immunosuppressive drugs should be administered daily, and if the administration is stopped, an immune rejection generally occurs. Accordingly, such an immunosuppressive drug should be administered for a long time, thereby inducing kidney toxicity, hepatotoxicity or high blood pressure. Moreover, since these drugs are not specific immunosuppressive drugs selectively acting only on immune cells that react only to an alloantigen of a graft, side effects such as opportunistic infection caused by non-specific immunosuppression or tumorigenesis such as lymphoma may occur. As still another immunosuppressive method, there is a method of administering monoclonal antibodies such as OKT3, Daclizumab or Basiliximab, but these antibodies are also non-specific immunosuppressive drugs and have a problem of opportunistic infection or tumorigenesis. Therefore, the development of new immunosuppressive methods without a problem of drug toxicity or opportunistic infection is required.

Meanwhile, human mesenchymal stem cells (MSCs) may be derived from various tissues, and are a potent candidate in cell-based transplantation or regenerative medication. The characteristics of MSCs such as migration to damaged tissue, an immunosuppressive function, self-renewal, and multipotency suggest the possibility of therapeutic application thereof. Today, about 500 clinical trials including injection or transplantation of MSCs are registered in clinicaltrials.gov. In addition, about 20% of the trials are associated with the immunosuppression of MSCs. The immunosuppressive property of MSCs is known, and although most of the phase 1 clinical trials do not show the problem of biological stability, insignificant results have been obtained at more advanced clinical phases.

Further, due to the absence of a culture method or protocols for the standardization of MSCs, even in the case of MSCs isolated from the same tissue of the same recipient, phenotypic and functional diversity have been observed. For this reason, a method for promoting or suppressing the function of MSCs by imparting specific conditions is required.

### [Disclosure]

### [Technical Problem]

Therefore, the inventors have made intensive efforts to develop a method for selecting mesenchymal stem cells having an excellent immunosuppressive property, and thus found the correlation between a particular immunosuppressive biomarker and immunosuppressive activity in mesenchymal stem cells in which SOCS expression/activity is downregulated, and also found that, when the expression of these biomarkers increases, the immunosuppressive activity of the mesenchymal stem cells is further improved than when only SOCS is downregulated. Thus, the present invention was completed.

Accordingly, the present invention is directed to providing a method for selecting high-efficiency mesenchymal stem cells for treating an immune disease, which includes measuring a level of a particular immunosuppressive biomarker (IDO, CXCL9, CXCL10, CXCL11, CXCL12, PTGES, CXCR4, CXCR7, VCAM1, ICAM1, ICAM2, TNF-alpha, B7-H1, TRAIL, or Fas Ligand) in mesenchymal stem cells in which the expression or activity of SOCS is downregulated, and high-efficiency mesenchymal stem cells selected thereby.

However, technical problems to be solved in the present invention are not limited to the above-described problems, and other problems which are not described herein will be fully understood by those of ordinary skill in the art from the following descriptions.

### [Technical Solution]

The present invention provides a method for selecting high-efficiency mesenchymal stem cells for treating an immune disease, which includes measuring a level of a particular immunosuppressive biomarker in mesenchymal stem cells in which the expression or activity of SOCS is downregulated.

According to an exemplary embodiment of the present invention, the method includes the following steps:
(a) culturing mesenchymal stem cells and treating the cells with a SOCS expression or activity inhibitor;
(b) measuring an expression level of an immunosuppressive biomarker in the mesenchymal stem cells in which the expression or activity of SOCS is downregulated; and
(c) determining the cells in which the expression level is downregulated, compared with a control which is not treated with a SOCS expression or activity inhibitor, as high-efficiency mesenchymal stem cells for treating an immune disease.

According to another exemplary embodiment of the present invention, the method further includes a step of culturing mesenchymal stem cells at a high density between the step (a) and the step (b).

According to still another exemplary embodiment of the present invention, the immunosuppressive biomarker is one or more selected from the group consisting of indoleamine 2,3-dioxygenase (IDO), C-X-C motif ligand 9 (CXCL9), C-X-C motif ligand 10 (CXCL10), C-X-C motif ligand 11 (CXCL11), C-X-C motif ligand 12 (CXCL12), Prostaglandin E synthase (PTGES), C-X-C chemokine receptor type 4 (CXCR4), C-X-C chemokine receptor type 7 (CXCR7), Vascular Cell Adhesion Molecule 1 (VCAM1), InterCellular Adhesion Molecule 1 (ICAM1), InterCellular Adhesion Molecule 2 (ICAM2), TNF-alpha, B7 homolog 1 (B7-H1), TNF-Related Apoptosis-Inducing Ligand (TRAIL) and Fas Ligand.

According to yet another exemplary embodiment of the present invention, the high efficiency is related to an immunosuppressive activity.

According to yet another exemplary embodiment of the present invention, the mesenchymal stem cells are derived from any one selected from the group consisting of umbilical cord, umbilical cord blood, bone marrow, fat, muscle, Wharton's jelly, nerve, skin, amniotic membrane, chorion, decidua and placenta.

According to yet another exemplary embodiment of the present invention, the immune disease is graft-versus-host disease, rejection in organ transplantation, humoral rejection, an autoimmune disease or an allergic disease.

According to yet another exemplary embodiment of the present invention, the autoimmune disease is Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

According to yet another exemplary embodiment of the present invention, the SOCS expression inhibitor is selected from the group consisting of small interference RNA (siRNA), short hairpin RNA (shRNA), microRNA (miRNA), peptide nucleic acids (PNA) and antisense oligonucleotide, which are specific for a SOCS gene.

According to yet another exemplary embodiment of the present invention, the SOCS activity inhibitor is selected from the group consisting of an antibody, an aptamer and an antagonist specific for a SOCS protein.

According to yet another exemplary embodiment of the present invention, the expression level of the biomarker in the step (b) is measured using western blotting, antibody immunoprecipitation, ELISA, mass spectrometry, RT-PCR, competitive RT-PCR, real-time RT-PCR, RNase protection assay (RPA), northern blotting or a DNA chip.

In addition, the present invention provides high-efficiency mesenchymal stem cells for treating an immune disease, which are selected by the above-described method.

According to yet another exemplary embodiment of the present invention, the immune disease is graft-versus-host disease, rejection in organ transplantation, humoral rejection, an autoimmune disease or an allergic disease.

According to yet another exemplary embodiment of the present invention, the high efficiency is related to an immunosuppressive activity.

According to yet another exemplary embodiment of the present invention, the mesenchymal stem cells are derived from umbilical cord, umbilical cord blood, bone marrow, fat, muscle, Wharton's jelly, nerve, skin, amniotic membrane or placenta.

According to yet another exemplary embodiment of the present invention, the mesenchymal stem cells may be derived from autologous, xenogeneic or allogeneic cells.

In addition, the present invention provides a pharmaceutical composition for treating an immune disease, which contains the high-efficiency mesenchymal stem cells.

In addition, the present invention provides a pharmaceutical preparation for treating graft-versus-host disease, which contains the high-efficiency mesenchymal stem cells.

In addition, the present invention provides a method for treating an immune disease such as graft-versus-host disease, which includes administering the high-efficiency mesenchymal stem cells to a subject.

In addition, the present invention provides a use of the high-efficiency mesenchymal stem cells for treating an immune disease such as graft-versus-host disease.

### [Advantageous Effects]

The present invention suggests that a particular combination of immunosuppressive biomarkers (IDO, CXCL9, CXCL10, CXCL11, CXCL12, PTGES, CXCR4, CXCR7, VCAM1, ICAM1, ICAM2, TNF-alpha, B7-H1, TRAIL, and Fas Ligand) is able to be used as a biomarker for selecting mesenchymal stem cells having excellent immunosuppressive activity with respect to immune-associated diseases including graft-versus-host disease in mesenchymal stem cells in which the expression/activity of SOCS is downregulated.

Accordingly, the present invention can provide a useful method capable of obtaining functionally excellent mesenchymal stem cells having immune response regulatory activity for clinical treatment of various immune diseases including graft-versus-host disease and an autoimmune disease, and can be effectively used as a therapeutic method for an immune disease.

In addition, prevention and treatment of graft-versus-host disease using the mesenchymal stem cells selected according to the present invention show a higher therapeutic possibility of allogeneic stem cell transplantation.

### [Description of Drawings]

FIG. 1A shows the result of red fluorescent staining confirming that SOCS protein expression is downregulated by treating MSCs with shRNA targeting SOCS.
FIG. 1B shows the western blotting result confirming that SOCS protein expression is downregulated by treating MSCs with shRNA targeting SOCS.
FIG. 2 shows the result confirming the immunosuppressive characteristic of MSCs in which SOCS is downregulated in an in vivo graft-versus-host disease animal model.

### [Modes of the Invention]

The present invention provides a method for selecting high-efficiency mesenchymal stem cells for treating an immune disease, which includes measuring a level of a particular immunosuppressive biomarker in mesenchymal stem cells in which the expression or activity of SOCS is downregulated.

In the present invention, SOCS protein belongs to the negative feedback regulator group of cytokine signaling, and has been known to include Janus kinase/signal transducers and activators of the transcription (JAK/STAT) pathway.

The type of SOCS is not limited, and may be, for example, a cytokine inducible SH2-containing protein (CISH), SOCS1, SOCS2, SOCS3, SOCS4, SOCS5, SOCS6 or SOCS7, and most preferably SOCS1 or SOCS3.

In the present invention, an immunosuppressive biomarker is not limited, but may be, for example, one or more selected from the group consisting of indoleamine 2,3-dioxygenase (IDO), C-X-C motif ligand 9 (CXCL9), C-X-C motif ligand 10 (CXCL10), C-X-C motif ligand 11 (CXCL11), C-X-C motif ligand 12 (CXCL12), Prostaglandin E synthase (PTGES), C-X-C chemokine receptor type 4 (CXCR4), C-X-C chemokine receptor type 7 (CXCR7), Vascular Cell Adhesion Molecule 1 (VCAM1), InterCellular Adhesion Molecule 1 (ICAM1), InterCellular Adhesion Molecule 2 (ICAM2), TNF-alpha, B7 homolog 1 (B7-H1), TNF-Related Apoptosis-Inducing Ligand (TRAIL) and Fas Ligand.

Here, it is known that PTGES serves to synthesize PGE2, VCAM1 serves to bind cells, CXCR7 serves as a receptor of a chemokine to recruit stem cells to an inflammatory region, and ICAM1 is involved in attachment and migration of inflammatory cells.

In addition, the selection method of the present invention may include (a) culturing mesenchymal stem cells and treating the cells with a SOCS expression or activity inhibitor; (b) measuring an expression level of an immunosuppressive biomarker in mesenchymal stem cells in which SOCS expression or activity is downregulated; and (c) determining the cells in which the expression level is downregulated compared with a control group which is not treated with a SOCS expression or activity inhibitor as high-efficiency mesenchymal stem cells for treating an immune disease.

In the present invention, the method may further include culturing mesenchymal stem cells at a high density between the step (a) and the step (b), and the high-density culture may be cultured at a density of 5,000 to 20,000 cells/cm².

The "high efficiency" used herein means effective efficiency in treatment of an immune-associated disease due to excellent immunosuppressive activity of mesenchymal stem cells.

The "mesenchymal stem cells (MSCs)" used herein refers to multipotent stem cells having an ability to differentiate into various mesodermal cells including bone, cartilage, adipose and muscular cells or ectodermal cells such as nerve cells. The MSCs are preferably derived from any one selected from the group consisting of umbilical cord, umbilical cord blood, bone marrow, fat, muscle, nerve, skin, amniotic membrane, chorion, decidua and placenta. In addition, the MSCs may be derived from a human, a fetus, or a non-human mammal. The non-human mammal is more preferably a canine animal, a feline animal, a simian animal, a cow, sheep, a pig, a horse, a rat, a mouse or a guinea pig, but the origin thereof is not limited.

The "immune disease" used herein is not limited as long as it is a disease caused by an immunomodulatory disorder, and may be, for example, graft-versus-host disease, rejection in organ transplantation, humoral rejection, an autoimmune disease or an allergic disease.

Here, the type of the autoimmune disease is not limited, and may be Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

Here, the type of the allergic disease is not limited, and may be anaphylaxis, allergic rhinitis, asthma, allergic conjunctivitis, allergic dermatitis, atopic dermatitis, contact dermatitis, urticaria, pruritus, insect allergy, food allergy or drug allergy.

In the present invention, the SOCS expression inhibitor is not limited as long as it specifically binds to a SOCS gene/mRNA to inhibit expression, and may be, for example, small interference RNA (siRNA) specific for a SOCS gene/mRNA, short hairpin RNA (shRNA), microRNA (miRNA), peptide nucleic acids (PNAs) or an antisense oligonucleotide.

In the present invention, the SOCS activity inhibitor is not limited as long as it binds to/competes with a SOCS protein to inhibit the activity, and may be, for example, an antibody, an aptamer and an antagonist specific for a SOCS protein.

In the present invention, a method for measuring an expression level of a biomarker is not limited, and the protein expression may be measured using, for example, western blotting, antibody immunoprecipitation, ELISA, mass spectrometry, and the mRNA expression may be measured using RT-PCR, competitive RT-PCR, real-time RT-PCR, RNase protection assay (RPA), northern blotting or a DNA chip.

In addition, the present invention provides high-efficiency MSCs for treating an immune disease, which are selected by the above-described method. There is no limit to the origin of the MSCs, and thus the MSCs may be derived from autologous, xenogeneic or allogeneic cells.

In addition, the present invention provides a pharmaceutical composition/pharmaceutical preparation for treating an immune disease, which contains the high-efficiency MSCs.

The "pharmaceutical composition" used herein may further include a component such as a conventional therapeutically active component, any of other additives, or a pharmaceutically acceptable carrier. Examples of the pharmaceutically acceptable carriers are saline, sterile water, Ringer's solution, buffered saline, a dextrose solution, a maltodextrin solution, glycerol, and ethanol.

The composition may be used by being formulated in the form of an oral preparation such as a powder, granules, a tablet, a capsule, a suspension, an emulsion, a syrup or an aerosol, a preparation for topical use, a suppository, or a sterile injection solution according to a conventional method.

A range of the "dose" used herein may be adjusted variously according to a patient's body weight, age, sex, health condition, diet, administration time, administration route, excretion rate, and severity of a disease, which is obvious to those of ordinary skill in the art.

The "subject" used herein means a subject in need of treatment, more specifically, a mammal such as a human, a non-human primate, a mouse, a rat, a dog, a cat, a horse or a cow.

The "pharmaceutically effective amount" used herein may be determined according to parameters including the type and severity of a disease for which administration of a drug is needed, the age and sex of a patient, the sensitivity to a drug, an administration time, an administration route, an excretion rate, a treatment period, a simultaneously-used drug, and other parameters well known in the medical field, and it is an amount capable of obtaining the maximum effect without side effects in consideration of all parameters, and may be easily determined by those of ordinary skill in the art.

There is no limit to the "administration route" as long as the composition of the present invention can reach target tissue. Examples of the administration routes include oral administration, intraarterial injection, intravenous injection, percutaneous injection, intranasal administration, transbronchial administration, and intramuscular administration. A daily dose may be about 0.0001 to 100 mg/kg, and preferably 0.001 to 10 mg/kg, and the administration is preferably performed once to several times a day.

Hereinafter, to help in understanding the present invention, exemplary examples will be suggested. However, the following examples are merely provided so that the present invention can be more easily understood, and not to limit the present invention.

### [Examples]

### Example 1: Experimental method

### 1-1. Isolation and culture of human tissue-derived MSCs

The experiment was approved (IRB No. 2011-10-134) by the Institutional Review Board (IRB) of the Samsung Medical Center, and all samples were collected with prior consent. The isolation of MSCs was performed by a conventionally known method. The isolated cells were seeded at a density of 2×10³ cells/cm² in Dulbecco's Modified Eagle's Medium (DMEM; Invitrogen-Gibco, Rockville, MD) containing 10% fetal bovine serum (FBS, Invitrogen-Gibco) and 100 U/mL penicillin/streptomycin (Invitrogen-Gibco), and cultured under conditions of 37 °C and 5% CO₂.

### 1-2. shRNA transfection

To downregulate SOCS1 expression, adipose tissue-derived MSCs (AT-MSC) were treated with an adenovirus expressing SOCS1 shRNA and a red fluorescent protein (RFP). Specifically, the shRNA sequence targeting SOCS1 was cloned in a human U6 promoter and a TagRFP marker gene-containing shuttle vector (pO6A5-U6-mPGK-TagRFP), thereby preparing a pO6A5-U6-shSOCS1-mPGK-TagRFP expression vector, and a U6-shRNA-SV40-pA region in the shuttle vector was recombined and transferred to a BAC vector. The recovered recombinant adenovirus (Ad5-U6-shSOCS1-mPGK-TagRFP) was proliferated in HEK-293 cells, and a protein transduction domain (PTD), HP4, was purchased from Peptron Inc. To transfect adenovirus particles into MSCs, adenovirus particles were treated together with HP4 (100 nM) at multiplicity of infection (MOI) 100, and cultured in a non-serum medium at room temperature for 30 minutes. Afterward, the cultured cells were washed with PBS and cultured with Ad-RFP-shSOCS1 and HP4 preparation, and after two hours, the cells were rinsed with PBS, and then cultured in a serum-containing medium. Selected MSCs were confirmed by fluorescence observation with an RFP and western blotting.

### 1-3. Immunoblotting

Cells were washed with cold PBS (Gibco-Invitrogen), and lysed using a protease inhibitor cocktail (Thermo Fisher Scientific, Rockford, IL, USA) in 300 µl of a cold RIPA buffer [1% Triton X-100, 150 mM NaCl, 0.1% sodium dodecyl sulfate (SDS), and 1% sodium deoxycholate-containing 50 mM Tris-HCl, pH 7.5]. The cell lysate was centrifuged at 4 °C for 10 minutes at 3000g, a supernatant was collected, and a protein concentration was determined using a BCA protein assay kit (Thermo Fisher Scientific). For electrophoresis, 30 µg of a protein was lysed in a sample buffer (14.4 mM β-mercaptoethanol, 25% glycerol, 2% SDS, and 0.1% bromophenol blue-containing 60 mM Tris-HCl, pH 6.8), boiled for 5 minutes, and isolated in a 10% SDS reducing gel. The isolated protein was transferred to a polyvinylidene difluoride (PVDF) membrane (Amersham Biosciences, Little Chalfont, Buckinghamshire, UK) using a trans-blot system (Gibco-Invitrogen). The PVDF membrane was blocked with TBS (150 mM NaCl-containing 10 mM Tris-HCl, pH 7.5) containing 5% skim milk powder (BD Sciences, CA, USA) for 1 hour at room temperature, washed with TBS three times, and incubated overnight with primary antibodies (all antibodies were diluted at 1:1000) in TBST (150 mM NaCl, and 0.02% Tween-20-containing 10 mM Tris, pH 7.5) containing 3% skim milk powder at 4 °C. The following day, blots were washed with TBST three times, and incubated with HRP-conjugated secondary antibodies (diluted at 1:2000 or 1:5000) in 3% skim-milk-powder-containing TBST at room temperature for 1 hour. The secondary-antibody-conjugated blots were washed with TBST three times, and the proteins were visualized using an ECL detection system (Amersham Biosciences).

### 1-4. Graft-versus-host disease (GVHD) animal model

300 cGy of total body irradiation was performed on 8- to 9-week-old NOD/SCID immunodeficient mice (Jackson Laboratories, Bar Harbor, ME), and after 24 hours, human peripheral blood mononuclear cells were intravenously administered. Specifically, 2×10⁷ human peripheral blood mononuclear cells were injected together with 1×10⁶ MSCs in each mouse. Afterward, the same number of MSCs were repeatedly injected 7 days after the injection.

### Example 2: Experimental result

### 2-1. Downregulation of SOCS expression

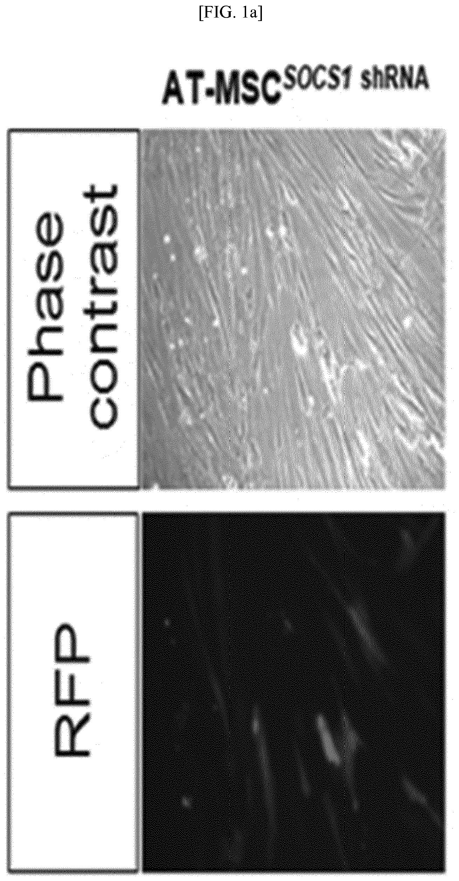

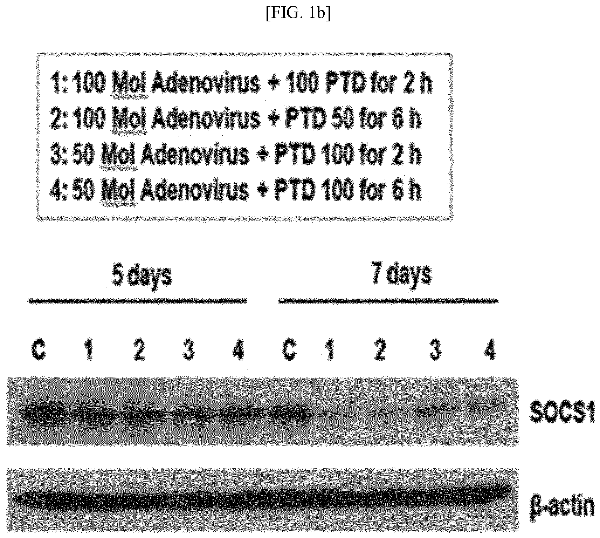

The downregulation of SOCS expression in MSCs was confirmed by the transduction of adipose-tissue-derived MSCs (AT-MSC) with a shRNA-expressing adenovirus targeting SOCS1. As a result, as shown in FIGS. 1A and 1B, from the results of RFP labeling (FIG. 1A) and western blotting (FIG. 1B), it can be confirmed that SOCS1 protein expression in MSCs was significantly downregulated.

### 2-2. Evaluation of in vivo immunosuppressive activity of SOCS-downregulated MSCs

It was confirmed whether the SOCS-downregulated MSCs obtained in Example 2-1 actually exhibited an immunosuppressive property in a GVHD animal model.

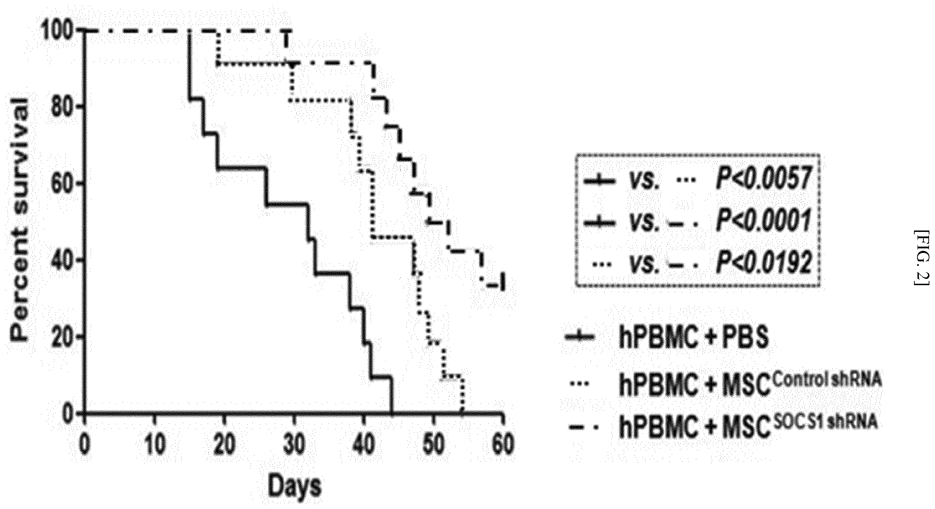

As a result, as shown in FIG. 2, it can be seen that an MSC (red line)-treated group in which SOCS was downregulated with shRNA had a considerably increased survival rate (immunosuppressive property), compared with a control.

### 2-3. Discovery of additional immunosuppressive biomarker in SOCS-downregulated MSCs

To examine the influence of the downregulation of SOCS expression on the gene expression in MSCs, before and after the treatment with a SOCS expression inhibitor (shRNA), gene expression profiles of MSCs were compared. As a result, it was confirmed that there was no significant difference in morphological changes before and after treatment, but there were significant differences in gene expression profiles.

Actually, the expression of various genes was increased in MSCs treated with a SOCS expression inhibitor (shRNA), and it can be seen that, among these genes, IDO, CXCL9, CXCL10, CXCL11, CXCL12, PTGES, CXCR4, CXCR7, VCAM1, ICAM1, ICAM2, TNF-alpha, B7-H1, TRAIL, and Fas Ligand genes are involved in the immunosuppression of MSCs.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The present invention can provide a method useful for acquiring functionally excellent MSCs having an ability to control immune reactions for clinical treatment of various immune diseases including graft-versus-host diseases and autoimmune diseases, and effectively used in therapy of immune diseases.

## Claims

1. A method of selecting high-efficiency mesenchymal stem cells (MSCs) for treating an immune disease, comprising:
measuring a level of an immunosuppressive biomarker in MSCs in which the expression or activity of a suppressor of cytokine signaling (SOCS) is downregulated.

2. The method according to claim 1, comprising the following steps:
(a) culturing mesenchymal stem cells and treating the cells with a SOCS expression or activity inhibitor;
(b) measuring an expression level of an immunosuppressive biomarker in the mesenchymal stem cells in which the expression or activity of SOCS is downregulated; and
(c) determining the cells in which the expression level is downregulated, compared with a control which is not treated with a SOCS expression or activity inhibitor, as high-efficiency mesenchymal stem cells for treating an immune disease.

3. The method according to claim 2, further comprising a step of culturing mesenchymal stem cells at a high density between the step (a) and the step (b).

4. The method according to claim 1, wherein the immunosuppressive biomarker is one or more selected from the group consisting of indoleamine 2,3-dioxygenase (IDO), C-X-C motif ligand 9 (CXCL9), C-X-C motif ligand 10 (CXCL10), C-X-C motif ligand 11 (CXCL11), C-X-C motif ligand 12 (CXCL12), Prostaglandin E synthase (PTGES), C-X-C chemokine receptor type 4 (CXCR4), C-X-C chemokine receptor type 7 (CXCR7), Vascular Cell Adhesion Molecule 1 (VCAM1), InterCellular Adhesion Molecule 1 (ICAM1), InterCellular Adhesion Molecule 2 (ICAM2), TNF-alpha, B7 homolog 1 (B7-H1), TNF-Related Apoptosis-Inducing Ligand (TRAIL) and Fas Ligand.

5. The method according to claim 1, wherein the high efficiency is related to an immunosuppressive property.

6. The method according to claim 1, wherein the MSCs are derived from any one selected from the group consisting of umbilical cord, umbilical cord blood, bone marrow, fat, muscle, Wharton's jelly, nerve, skin, amniotic membrane, chorion, decidua and placenta.

7. The method according to claim 1, wherein the immune disease is graft-versus-host disease, rejection in organ transplantation, humoral rejection, an autoimmune disease or an allergic disease.

8. The method according to claim 7, wherein the autoimmune disease is Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

9. The method according to claim 2, wherein the SOCS expression inhibitor is selected from the group consisting of small interference RNA (siRNA), short hairpin RNA (shRNA), microRNA (miRNA), peptide nucleic acids (PNA) and antisense oligonucleotide, which are specific for a SOCS gene.

10. The method according to claim 2, wherein the SOCS activity inhibitor is selected from the group consisting of an antibody, an aptamer and an antagonist specific for a SOCS protein.

11. The method according to claim 2, wherein the expression level of the biomarker in the step (b) is measured using western blotting, antibody immunoprecipitation, ELISA, mass spectrometry, RT-PCR, competitive RT-PCR, real-time RT-PCR, RNase protection assay (RPA), northern blotting or a DNA chip.

12. High-efficiency mesenchymal stem cells (MSCs) for treating an immune disease, which are selected by the method of claim 1.

13. The cells according to claim 12, wherein the immune disease is graft-versus-host disease, rejection in organ transplantation, humoral rejection, an autoimmune disease or an allergic disease.

14. The cells according to claim 12, wherein the high efficiency is related to an immunosuppressive activity.

15. The cells according to claim 12, wherein the MSCs are derived from umbilical cord, umbilical cord blood, bone marrow, fat, muscle, nerve, skin, amniotic membrane or placenta.

16. The cells according to claim 12, wherein the MSCs are derived from autologous, xenogeneic or allogeneic cells.

17. A pharmaceutical composition for treating an immune disease, which comprises the high-efficiency mesenchymal stem cells (MSCs) according to claim 12.

18. A pharmaceutical preparation for treating graft-versus-host disease, which comprises the high-efficiency mesenchymal stem cells (MSCs) according to claim 12.

19. A method for treating an immune disease, comprising administering the high-efficiency mesenchymal stem cells (MSCs) according to claim 12 to a subject.

20. A use of the high-efficiency mesenchymal stem cells (MSCs) according to claim 12 to treat an immune disease.
